# EUROPEAN PATENT APPLICATION

(11) **EP 2 110 130 A1**
(43) Date of publication of application: **21.10.2009**
(21) Application number: 09005528.6
(22) Date of filing: 20.04.2009
(51) Int. Cl.: A61K 31/52, A61P 1/00, A61P 1/04

(54) **Pharmaceutical use of 6-mercaptopurine**

(30) Priority: 18.04.2008 US 46152 P
(71) Applicant: Teva Pharmaceutical Industries Ltd., 49131 Petah Tiqva (IL)
(72) Inventor: Rosenberger, Vered, Modiin 71700 (IL); Kolatch, Brenda, Jerusalem 95450 (IL); Hotovely-Salomon, Anna, Jerusalem 96400 (IL); Marshall, Susan Linda Dr., Jerusalem 92544 (IL)
(74) Representative: King, Lawrence

(57) **Abstract**

Use of 6-mercaptopurine for the manufacture of a medicament for treating mild to moderate inflammatory bowel disease, preferably Crohn's disease or ulcerative colitis, wherein the medicament is an oral delayed release pharmaceutical composition.

## Description

### CROSS REFERENCE TO RELATED APPLICATIONS

This application claims the benefit of U.S. Provisional Patent Application Serial No. 61/046152, filed April 18, 2008, the contents of which are incorporated by reference herein, in their entirety.

### FIELD OF THE INVENTION

This invention relates to the treatment of inflammatory bowel disease by administering a delayed release formulation of 6-mercaptopurine.

### BACKGROUND OF THE INVENTION

Inflammatory bowel disease ("IBD"), including ulcerative colitis and Crohn's disease, affects many patients around the world. Crohn's disease is an idiopathic and chronic intestinal inflammation, affecting nearly 1% of the population of the western world, with average age of diagnosis between 15 and 30 years. In patients with small intestinal disease, the terminal ileum is involved in 90% of cases. Characterized by flare-ups and remissions, Crohn's disease causes damage across the entire thickness of the intestinal mucosa, with segmental "skip" lesions alternating with healthy tissue. Typical features of Crohn's disease include persistent diarrhea, right lower-quadrant abdominal pain, low grade fever, and weight loss.

At the cellular level of the intestine, several mechanisms are reported to be involved in antigen presentation. It is theorized that an autoimmune component plays a central role in the development of Crohn's disease. The proximity of the ileum to immune-rich tissue, the Peyer's patches that serve as the antigen presentation arm of the intestines, may be the reason for the high degree of terminal ileum involvement in small bowel Crohn's disease. It may also be the reason why the mucosal damage seen in Crohn's disease does not spread beyond the gastrointestinal tract.

Apparently, in Crohn's disease, the aberrant immune response is regulated by Type 1 T helper cells, resulting from the proliferation and differentiation of T cells into effector T cells, producing cytokines that magnify the immune response. Such cytokines may include interferon ("IFN")-γ, interleukin ("IL")-2, and IL-18. Due to defective apoptosis, the reaction of the immune response does not terminate, and results in an ongoing exaggerated T-cell response. A broad cascade of inflammatory mediators such as tumor necrosis factor ("TNF")-α are quantifiable.

The main goal of treatment for Crohn's disease has always been the induction of symptomatic improvement (i.e., "clinical response") and maintenance of such improvement or, at best, "remission". The assessment of Crohn's disease severity and evidence for clinical "response" or "remission" in Crohn's disease are indicated by the Crohn's Disease Activity Index ("CDAI"), the widely-accepted gold standard for quantifying the symptoms of CD patients. The CDAI consists of eight factors, each summed after adjustment with a weighting factor (e.g., clinical symptoms, complications, laboratory criteria, and a physician's global assessment of a patient's well-being). "Remission" is typically defined as a CDAI score of <150, while "response" is defined as a drop of 100, or at least 70 points, in the baseline CDAI score. The standard method for calculating CDAI can be found in: Best W, Becktel J, Singleton J, Kern F. Development of a Crohn's disease activity index: National cooperative Crohn's disease study. Gastroenterology. 1976; 70: 439-44.

Recently, the severity of mucosal inflammation, as assessed by endoscopy, has been touted an additional mainstay parameter for assessment in clinical trials. The importance of mucosal tissue healing in inflammatory bowel disease, in general, has become clinically relevant in light of recent reports correlating disease activity with a patient's overall risk of developing colorectal cancer.

The Crohn's Disease Endoscopic Index of Severity ("CDEIS") is a commonly-accepted clinical measure of mucosal healing in Crohn's disease. The CDEIS is based upon the presence or the absence of 4 types of lesions: superficial ulcers, deep ulcers, ulcerated stenosis, or non-ulcerated stenosis, all of which are recorded in 5 different segments: terminal ileum, ascending colon, transverse colon, descending and sigmoid colon, and the rectum. In addition, for these 5 segments, the percentage of ulcerated colonic surface and the percentage of surface "affected by any Crohn's disease lesion" are indicated on a 10-cm visual analogue scale. The combination of values allows calculation of the severity score, which generally ranges between 0 and 30. Standard methods for calculating CDEIS can be found in: Guyatt G, Mitchell A, Irvine EJ, Singer J, Williams N, Goodacre R and Tompkins C. A new measure of health status for clinical trials in inflammatory bowel disease. Gastroenterology 1989;96:804-810. Mary JY, Modigliani R. Development and validation of an endoscopic index of the severity for Crohn's disease: a prospective multicentre study. Groupe d'Etudes Therapeutiques des Affections Inflammatoires du Tube Digestif (GETAID). Gut 1989; 30(7):983-989.

Another index of clinical improvement in CD is modification in the circulating blood levels of systemic immunologic cells, including serum and intracellular cytokines, T cell subsets and specifically, interferon-γ ("IFN- γ"). As a surrogate marker monitoring immunologic response, a reduction in IFN- γ levels, as measured using the highly sensitive enzyme-linked immunosorbent spot ("ELISPOT"), indicates improvement in the CD patient's immunological status. Fuss IJ, Neurath M, Boirivant M, Klein JS, de la Motte C, Strong SA, Fiocchi C, Strober W. Disparate CD4+ lamina propria (LP) lymphokine secretion profiles in inflammatory bowel disease. Crohn's disease LP cells manifest increased secretion of IFN-gamma, whereas ulcerative colitis LP cells manifest increased secretion of IL-5. Journal of Immunology. 1996 Aug 1; 157(3):1261-70.

Additionally, the measurement of circulating blood levels of CRP (C-reactive protein) and ESR (erythrocyte sedimentation rate) assess a patient's general inflammatory status, with a reduction in these levels, indicative of symptomatic improvement. Similarly, the IBDQ ("Inflammatory bowel disease questionnaire"), a 32 item validated questionnaire assessing a patient's IBD symptoms, general well-being and mood, is typically used as a research tool to evaluate a patient's quality-of-life, with a score of ≥180 indicative of remission. Guyatt G, Mitchell A, Irvine EJ, Singer J, Williams N, Goodacre R and Tompkins C. A new measure of health status for clinical trials in inflammatory bowel disease. Gastroenterology 1989;96:804-810.

6-MP is a synthetic analogue of natural purine bases. After absorption into the body, it is presumably transformed into nucleotides which interfere with nucleic acid biosynthesis, especially in the active S phase. As such, it used to slow the growth of cancerous cells.

6-MP received FDA approval for remission induction and maintenance therapy of childhood acute lymphatic leukemia in 1953. 6-MP is therefore indicated as a monotherapy and as part of combination therapies for treating acute lymphocytic leukemia in both adults and children (Physician's Desk Reference 57th Edition, 2003, page 1615-1618). A standard 6-MP (immediate release) 50 mg tablet is described in Physician's Desk Reference 57th Edition, 2003, page 1615-1618 and is sold in the United States under the brand name PURINETHOL^{®}. 6-MP also exhibits immunosuppressive properties. While it is not officially indicated for diseases where treatment with immunosuppressive agents is beneficial, 6-MP has been widely used for several such conditions. It has reportedly been prescribed off-label for the treatment of inflammatory bowel diseases, such as Crohn's disease and ulcerative colitis, for over thirty years. In this context, lower doses (1.0 - 2.0 mg/kg) of the commercially available 6-MP immediate release tablet have been employed.

6-MP and its parent drug, azathioprine, have been reported to be slow acting, usually requiring 3-6 months to be effective. Kim et. al, "Optimum Duration Of Treatment With 6-Mercaptopurine For Crohn's Disease," American Journal of Gastroenterology, 94:3254-3257 (1999). They are usually used to maintain remission in patients with refractory or corticosteroid-dependent forms of IBD. D'Haens et al., "Endoscopic and Histologic Healing of Crohn's (Ileo-) Colitis with Azathioprine," Gastrointestinal Endoscopy, 50:667-671(1999).

Because it is known that 6-MP is slow-acting and requires at least 12 weeks to show any benefit, it is typically added to initial steroids to ease steroid tapering in remission induction and is continued as maintenance, often for years. AZA and 6-MP act through inhibition of nucleotide biosynthesis and purine nucleotide interconversion. Reportedly, when administered, 6-MP, itself inactive, is broken down by several competing enzymatic pathways to active metabolites and inactive end products. The active metabolites serve as purine antagonists, interfering with DNA and RNA synthesis and chromosomal replication, leading to diminished proliferation of rapidly dividing cells. Apparently the active metabolites specifically block gene activation of effective lymphocyte clones. In the circulation, killer cell activity is reduced, and in the mucosal lamina propria, the absolute number of plasmocytes is lowered.

### SUMMARY OF THE INVENTION

The present invention provides a method of treating inflammatory bowel disease that provides local mucosal healing following treatment with an oral delayed-release 6-MP pharmaceutical formulation despite very little systemic absorption of 6-MP.

In one embodiment, the invention provides a method of treating inflammatory bowel disease comprising administering an oral delayed release pharmaceutical composition comprising 6-MP to patients having mild to moderate inflammatory bowel disease. In certain embodiments, the inflammatory bowel disease is Crohn's disease or ulcerative colitis.

In certain embodiments, the present invention comprises administering a delayed release pharmaceutical composition comprising 6-MP to a patient in need of such administration where the delayed release pharmaceutical composition comprises less than 50 mg of 6-MP. In certain embodiments, the delayed release pharmaceutical composition comprises about 40 mg 6-MP.

In another embodiment, the present invention provides a method of treating inflammatory bowel disease with a non-systemically absorbed oral delayed release 6-MP pharmaceutical composition wherein no more than 15 ng/ml of 6-MP is present in the blood after about 9 hours of dosing less than 50 mg of 6-MP under fast condition. In certain embodiments, the blood level of 6-MP is undetectable. Despite such low blood levels of 6-MP, administration of delayed release pharmaceutical composition comprising 6-MP according to the present invention results in mucosal healing, clinical efficacy, improvement of immunological status measured by improvement of immunological markers or a combination thereof. Unlike treatment with traditional 6MP (immediate release), treatment with delayed release 6MP does not have to be discontinued or interrupted or dosage reduced due to deleterious changes in liver enzymes and/or white blood cell counts. Thus, the present invention provides these benefits along with a greatly diminished incidence of side effects.

### DETAILED DESCRIPTION OF THE INVENTION

As used herein, "mild to moderate Crohn's disease" is exemplified by a CDAI value of about 220 to about 400.

As used herein, "patient" generally refers to a human.

As used herein, a "delayed release 6-MP pharmaceutical composition" or a "delayed release pharmaceutical composition comprising 6-MP" refers to a pharmaceutical composition comprising 6-MP where release of 6-MP occurs after passage of the pharmaceutical composition through the stomach. Preferably, the pharmaceutical composition is enterically coated. Preferably, the enteric coating imparts a delay in the release in the 6-MP following oral administration of the pharmaceutical composition such that release of 6-MP occurs after passage of the composition through the stomach. Optionally, the release of 6-MP occurs after at least about 1 hour, at least about 2 hours, or at least about 3 hours after passage of the composition through the stomach. Alternatively, the release of 6-MP occurs about 1 to about 3 hours or about 2 to about 3 hours after passage of the composition through the stomach. More preferably, the release of 6-MP occurs about 5 to about 6 hours after ingestion.

In one embodiment, the present invention provides a method of treating inflammatory bowel diseases such as Crohn's disease or ulcerative colitis comprising administering an oral delayed release 6-MP pharmaceutical composition such that release of 6-MP occurs after passage of the pharmaceutical composition through the stomach, wherein blood levels of 6-MP do not rise above 15 ng/ml but nevertheless administration of the 6-MP pharmaceutical composition results in mucosal healing, clinical efficacy, improvement of immunological markers or combination thereof.

In a preferred embodiment, the delayed release 6-MP pharmaceutical composition is administered to a patient having active inflammatory bowel disease. Most preferably, the delayed release 6-MP pharmaceutical composition is administered to a patient having mild to moderate inflammatory bowel disease.

In a preferred embodiment, a delayed release pharmaceutical composition comprising 6-MP is administered to a patient having mild to moderate Crohn's disease, as exemplified by a CDAI of less than 400.

In another embodiment, the present invention provides a method of treating inflammatory bowel disease with a non-systemically absorbed oral delayed release 6-MP pharmaceutical composition wherein no more than 15 ng/ml of 6-MP is present in the blood after about 9 hours of dosing less than 50 mg of 6-MP under fast condition. Preferably, no more than 10 ng/ml of 6-MP is present in the blood after about 9 hours of dosing 40 mg of 6-MP under fast condition of at least 2 hours. In certain embodiments, the present invention provides a method of treating inflammatory bowel disease comprising administering to a patient having inflammatory bowel disease a delayed release pharmaceutical composition comprising 6-MP where such administration results in a plasma Cₘₐₓ of 6-MP of not more than 15 ng/ml, not more than 10 ng/ml, not more than 9 ng/ml, not more than 8 ng/ml, not more than 7 ng/ml, not more than 6 ng/ml, not more than 5 ng/ml, not more than 4 ng/ml, not more than 3 ng/ml, not more than 2 ng/ml, or not more than 1 ng/ml. In certain embodiments, the plasma Cₘₐₓ of 6-MP is 8-10 ng/ml, 7-9 ng/ml, 6-8 ng/ml, 5-7 ng/ml, 4-6 ng/ml, 3-5 ng/ml, 2-4 ng/ml, 1-3 ng/ml, 0.5-2 ng/ml, or 0.1-1 ng/ml. In certain embodiments, the plasma Cₘₐₓ of 6-MP is about 10 ng/ml, about 9 ng/ml, about 8 ng/ml, about 7 ng/ml, about 6 ng/ml, about 5 ng/ml, about 4 ng/ml, about 3 ng/ml, about 2 ng/ml, or about 1 ng/ml.

In certain embodiments, the present invention provides a method of treating inflammatory bowel disease comprising administering to a patient having inflammatory bowel disease a delayed release oral pharmaceutical composition comprising 6-MP where such administration results in an average plasma Cₘₐₓ of 6-MP of about 1% to about 10%, preferably of about 1% to about 5%, more preferably of about 1% to about 2%. Optionally the average plasma Cₘₐₓ of 6-MP is less than 10%, preferably less than 5%, more preferably less than 2%.

In certain embodiments, the present invention provides a method of treating inflammatory bowel disease comprising administering to a patient having inflammatory bowel disease a delayed release pharmaceutical composition comprising 6-MP where such administration results in an undetectable plasma Cₘₐₓ of 6-MP using an LC/MS/MS detection system with a lower limit of quantitation (LLOQ) of 2.0 ng/ml.

In certain embodiments, the present invention provides a method of treating inflammatory bowel disease comprising administering to a patient having inflammatory bowel disease a delayed release pharmaceutical composition comprising 6-MP where such administration results in an undetectable plasma Cₘₐₓ of 6-MP using an LC/MS/MS detection system with a limit of quantitation (LLOQ) of 0.5 ng/ml.

In another embodiment, the patient population is about 18 to about 75 years of age. Preferably, the patient population is about 20 to about 50 years of age. Optionally, the mean age of the patients is 31.0 ± 11.6 years.

Optionally, the patient's age at diagnosis is about 15 to about 35 years. Preferably, the mean age at diagnosis is 23.8 ± 7.8 years.

Suitable pharmaceutical compositions for use in the present invention delay the release of 6-MP in such a way that most of the 6-MP in the pharmaceutical composition is released in the intestines. U.S. Patent Application Nos. 2006/0008520 ("the '520 publication") and 2006/0009473 ("the '473 publication"), which are incorporated herein by reference in their entireties, disclose delayed release pharmaceutical compositions comprising 6-MP useful in this invention. In certain embodiments, delayed release is provided to a pharmaceutical composition comprising 6-MP by the use of an enteric coating. Preferably, the delayed release pharmaceutical composition comprising 6-MP contains an enteric coating ofmethacrylic acid copolymers NF, preferably EUDRAGIT^{®}.

Any dosage form containing 6-MP could be coated with enteric coatings. In another embodiment, the dosage form is powder, granules or pellets which are filled in a capsule.In one embodiment, the weight of the enteric coating applied to the tablets is about 100 mg. In a preferred embodiment, the delayed release pharmaceutical composition is an enterically coated tablet comprising 6MP, a potassium, sodium, magnesium, ammonium, or calcium salt of a pharmaceutically acceptable acid, layered (sprayed) on an acceptable pharmaceutical carrier powder selected from the group consisting of microcrystalline cellulose, lactose, starch, calcium phosphate,powdered cellulose, sorbitol, and sucrose or combination thereof. Preferably, the delayed release pharmaceutical composition is an enterically coated tablet comprising 40 mg 6-MP, microcrystalline cellulose or other pharmaceutical carrier having a size range of 1 to 800 micron, pharmaceutically acceptable acid exemplified by citric acid and pharmaceutically acceptable base exemplified by potassium hydroxide. Optionally, the delayed release pharmaceutical composition may further comprise PVP K30, colloidal silicon dioxide, potato starch, crospovidone and magnesium stearate. Lactose, starch, microcrystalline cellulose, calcium phosphate, powdered cellulose, sorbitol or sucrose are examples of pharmaceutically acceptable powders that can be used as pharmaceutical carriers for this invention. The pharmaceutical carriers refer to particles having a size range of 1 to 800 microns. Other pharmaceutical excipient powders are known in the art and may also be used. The base may be selected from any pharmaceutically acceptable base such as the hydroxide or carbonate salts of potassium, sodium, magnesium, ammonium or calcium. The acid may be selected from any pharmaceutically acceptable acid. Examples of such acids are acetic acid, ascorbic acid, benzoic acid, citric acid and tartaric acid. In another embodiment, the pharmaceutically acceptable acid is precoated in a slight stoichiometric excess onto the pharmaceutically acceptable carrier before it is used in the granulation with a basic organic solution of 6-MP.

In a particular embodiment, an organic solvent solution of 6-MP is spray granulated on to the powder so as to form a coating, preferably a uniform coating.

In a preferred embodiment, the delayed release pharmaceutical composition having improved solubility.

In certain embodiments of the present invention, the patient has not been treated with any steroid before the 6-MP treatment. Optionally, the patient has not been treated with any steroid within 1 month before the 6-MP treatment. In certain embodiments, the patient is not treated with any steroid during the 6-MP treatment.

In certain embodiments, the patient has not been treated with methotrexate, cyclosporine, anti TNF-α agent, and/or anti-integrin agent before the 6-MP treatment according to the present invention. In certain embodiments, the patient has not been treated with methotrexate, cyclosporine, anti TNF-α agent, and/or anti-integrin agent within 3 months before the 6-MP treatment. In certain embodiments, the patient is not treated with methotrexate, cyclosporine, anti TNF-α agent, or anti-integrin agent during the 6-MP treatment.

In certain embodiments, the total dose of 6-MP administered to the patient according to the methods and uses of the present invention is 140 mg/day or less. Optionally, the total dose of 6-MP is about 120 mg/day or less, about 60 mg/day or less or about 40 mg/day or less. More preferably, the total dose of 6-MP is less than about 80 mg/day, less than about 50 mg/day, about 45 mg/day or less or about 40 mg/day or less.

In certain embodiments, the dose of 6-MP administered according to the methods and uses of the present invention is about 45 mg/day, 40 mg/day, 35 mg/day, 30 mg/day, 25 mg/day, 20 mg/day, or 15 mg/day. In certain embodiments, the dose of 6-MP is about 1 to about 35, about 10 to about 35, or about 20 to about 35 mg/day. In certain embodiments, the dose of 6-MP is about 40 to about 45 mg/day, about 35 to about 40 mg/day, about 30 to about 35 mg/day, about 25 to about 30 mg/day, or about 20 to about 25 mg/day.

In certain embodiments, the dose of 6-MP administered according to the methods and uses of the present invention is about 0.02 to about 2 mg/kg patient weight per day, about 0.1 to about 1.8 mg/kg patient weight per day, or about 1 to about 1.5 mg/kg patient weight per day. preferably the dose of 6-MP is less than 2 mg/kg patient weight per day. More preferably, the dose of 6-MP is less than about 1.5 mg/kg, less than about 1 mg/kg, less than about 0.3 mg/kg patient weight per day. While not intending to be bound by any particular theory, the low blood levels of 6-MP can imply that dosing of delayed release pharmaceutical composition comprising 6-MP according to the present invention does not need to be adjusted according to patient weight.

Preferably, the delayed release 6-MP pharmaceutical composition is administered once per day. In certain embodiments, the delayed release 6-MP pharmaceutical composition is administered two or three times per day. More preferably, the delayed release 6-MP pharmaceutical composition is administered once daily prior to sleep, optionally while the patient is in the fasting state, as exemplified by at least 2 hours following a meal.

The present invention provides a method of dosing 6-MP to a patient with inflammatory bowel disease comprising administering a delayed release 6-MP pharmaceutical composition once per day or twice per day to the patient for a period of time greater than one day.

The present invention provides methods and uses of inducing remission in a patient with inflammatory bowel disease, comprising administering a delayed release 6-MP pharmaceutical composition. As used in the present invention, "Remission" is defined as a CDAI score of less than 150 points. Preferably, remission is reached within 12 weeks from the beginning of treatment. In certain embodiments, the delayed release 6-MP pharmaceutical composition is administered once per day or twice per day, preferably once per day. In certain embodiments, the delayed release 6-MP pharmaceutical composition comprises about 40 mg or more, as exemplified by about 60 mg to about 80 mg of 6-MP, preferably about 40 mg of 6-MP. In certain embodiments, the inflammatory bowel disease is Crohn's disease, particularly mild to moderate Crohn's disease, for example Crohn's disease as exemplified by patients with a CDAI score of less than about 400. In certain embodiments, the period of time to induce remission is about 1 week, about 2 weeks, about 3 weeks, about 4 weeks, about 5 weeks, about 6 weeks, about 7 weeks, about 8 weeks, about 9 weeks, about 10 weeks, about 11 weeks, about 12 weeks, about 13 weeks, about 14 weeks, about 16 weeks, or about 18 weeks.

The present invention provides a methods and uses of maintaining remission in a patient with inflammatory bowel disease comprising administering to a patient in remission a delayed release 6-MP pharmaceutical composition once per day for a period of time during which it is desired to maintain remission. Most preferably, remission is maintained during the use of the delayed release 6-MP pharmaceutical composition. Preferably maintaining remission can be achieved by once daily about 40 mg or less, more preferably about 20 mg or less said 6-MP.Altematively, treatment with a delayed release 6-MP pharmaceutical composition according to the methods of the present invention results in a response. A "response" is defined as a reduction of the CDAI score by 70 to 100 points from baseline (i.e., the CDAI score before the beginning of treatment). Preferably, the reduction of the CDAI score is about 150, 180 or more from baseline. Preferably, the response is achieved within 12 weeks from the beginning of treatment. Preferably, the reduction of the CDAI score is about 60 or more from baseline within 2 weeks or about 80 or more from baseline within 9 weeks.

Preferably, treating patients with Crohn's disease according to the invention reduces their symptoms and/or results in mucosal healing. Preferably, the treatment reduces the number and/or size of the ulcers in the patient. In certain embodiments, the methods and uses according to the present invention result in a reduction of CDEIS score, a measure of mucosal healing. In certain embodiments, the reduction of the CDEIS score is at least 5, 10, 12, 15, 20, 30, 40, 45, 50, 60, 70, 80, 90, or 100. In certain embodiments, the reduction of the CDEIS score is between 5-10, 10-20, 20-30, 30-40, 40-50, 50-60, 60-70, 70-80, 80-90, or 90-100. In certain embodiments, the reduction of the CDEIS score is about 5, about 10, about 20, about 30, about 40, about 50, about 60, about 70, about 80, about 90, or about 100.

Preferably, the reduction of the baseline CDEIS score (i.e., the CDEIS score measured before treatment) is achieved within 12 weeks from the beginning of the treatment. Preferably, the reduction in the baseline CDEIS score is 10%-20%, 20%-30%, 30%-40%, or 40%-50% following treatment. Preferably, the reduction in the baseline CDEIS score is about 10%, about 20%, about 30%, about 40%, or about 50% following treatment.

Preferably, treatment with the delayed release 6-MP pharmaceutical composition results improvement in the results of IFN-γ ELISPOT assay of a blood sample taken from the patient.

More preferably treatment with the delayed release 6-MP pharmaceutical composition results in a reduction of IFN-γ ELISPOT levels of about 50% or more within 12 weeks.

Preferably, treatment with the delayed release 6-MP pharmaceutical composition results in improvement in Erythrocyte Sedimentation Rate ("ESR"). Preferably, treatment with the delayed release 6-MP pharmaceutical composition results in improvement in C-Reactive Protein ("CRP"). Preferably, treatment with the delayed release 6-MP pharmaceutical composition results in improvement in Inflammatory Bowel Disease Questionnaire ("IBDQ") score. More preferably, the IBDQ score after treatment is about 180 or more.

Preferably, the treatment results in little or no side effects in the patients. Common side effects include, for example, low white blood cell count and liver function elevations, often requiring dose lowering or treatment discontinuation. For example, the side effects resulting from the methods of the present invention are preferably sufficiently low that most patients are able to finish a complete course of treatment according to the methods of the present invention.

In certain embodiments, less than 10%, less than 9%, less than 8%, less than 7%, less than 6%, less than 5%, less than 4%, less than 3%, less than 2%, or less than 1% of the 6-MP in the delayed release pharmaceutical composition comprising 6-MP is absorbed into the bloodstream.

Also within the scope of the present invention are methods of treatment using delayed release formulations of derivatives, prodrugs, metabolites, and pharmaceutically acceptable salts of 6-MP. Examples of prodrugs and metabolites, respectively, are azathioprine ("AZA") and 6-thioguanine nucleotides ("6TGN").

The present invention also provides the use of 6-MP for the manufacture of a medicament for treating inflammatory bowel disease, such as Crohn's disease, according to the disclosure herein. Thus, in one embodiment the present invention provides the use of 6-MP for the manufacture of a medicament for treating inflammatory bowel disease, such as Crohn's disease, where the medicament is a delayed release pharmaceutical composition. In another embodiment, the present invention provides the use of 6-MP for the manufacture of a medicament for inducing remission in a patient with active inflammatory bowel disease, such as Crohn's disease, where the medicament is a delayed release pharmaceutical composition. In another embodiment, the present invention provides the use of 6-MP for the manufacture of a medicament for maintaining remission in a patient with inflammatory bowel disease, such as Crohn's disease, where the medicament is a delayed release pharmaceutical composition.
[0010] In some embodiments of the above use, the delayed release pharmaceutical composition comprises less than 50 mg of 6-MP and provides a plasma Cₘₐₓ of 6-MP of less than 15 ng/ml. Preferably, the above use results in mucosal healing, clinical efficacy, improvement of immunological markers or a combination thereof.

In some embodiments of the above use, the delayed release pharmaceutical composition comprises about 45 mg, about 40 mg, about 35 mg, about 30 mg, about 25 mg, or about 20 mg, of 6-MP. In some embodiments of the above use, the delayed release pharmaceutical composition provides a plasma Cₘₐₓ of 6-MP of less than 15 ng/ml, less than 13 ng/ml, less than 10 ng/ml, less than 8 ng/ml, less than 7 ng/ml, less than 6 ng/ml, less than 5 ng/ml, less than 4 ng/ml, less than 3 ng/ml, less than 2 ng/ml, or less than 1 ng/ml.

### EXAMPLES

### Example 1: Clinical trial of the efficacy of a delayed release composition according to the present invention

The trial was conducted as an open-label, randomized, parallel group study to evaluate the clinical and immunological efficacy and the safety of a local delivery (delayed release) 6-MP pharmaceutical composition versus standard tablet PURINETHOL^{®} in non-steroid dependent patients with active Crohn's disease.

The efficacy outcome measures in this study were continuous measures generated by CDAI, CDEIS, and immunological (IFN-γ ELISPOT) or inflammatory markers (CRP and ESR). Clinical response or remission was measured by CDAI at each visit, up to 12 weeks. Intestinal tissue improvement (mucosal healing) was evaluated at 12 weeks by the CDEIS. The safety outcome measures included adverse events, physical examinations (BP, pulse, temperature) and clinical labs (mainly liver function tests), which were conducted at each visit.

The delayed release 6-MP pharmaceutical composition contained 40 mg of 6-MP and was prepared in accordance with the '520 publication and the '473 publication. The delayed release 6-MP pharmaceutical composition used in this study comprises a pH dependent enteric coating. The pharmaceutical composition is designed to pass through the stomach intact and to begin to release 6-MP two hours after leaving the stomach. Over 85% of the tablet contents will dissolve over a subsequent three hour period. As the normal transit time for the small intestine is three to four hours, the release of 6-MP is expected to occur in the ileum, preferably in the terminal ileum.

**Table 1. Formulation of the delayed release 6-MP pharmaceutical composition (per tablet)**

| **Ingredient** | **Weight (mg)** |
|---|---|
| Mercaptopurine | 40 |
| Microcrystalline Cellulose | 280 |
| Citric Acid anhydrate | 19.5 |
| Potassium hydroxide | 16.2 |
| PVP K30 | 10.4 |
| Colloidal Silicon Dioxide | 1.6 |
| Potato Starch | 24.4 |
| Crospovidone | 26.4 |
| Microcrystalline Cellulose | 91.6 |
| PVP K30 | 5.2 |
| Magnesium Stearate | 8.0 |
| Eudragit L100 | 77 |
| Triethyl citrate | 7.7 |
| Talc | 38.5 |

### Eligibility of Participating Patients:

| | |
|---|---|
| Ages Eligible for Study: | 18 Years to 75 Years |
| Genders Eligible for Study: | Both genders |

### Inclusion Criteria:

* Fifteen male or female, aged 18-75 years with moderate Crohn's disease (CDAI score ≥220 and ≤400 at screening) with or without adjunctive mesalamine treatment, 12 with involvement of the ileum and three without ileal involvement.
* Definitive diagnosis of active inflammatory Crohn's disease with fibrostenosing and/or fistulizing/perforating Crohn's disease types within the previous 6 months ruled out based on clinical and radiological or endoscopic or pathological findings.

### Exclusion Criteria:

* Body weight below 42.5 kg
* Subjects who have received either methotrexate, cyclosporine, or anti TNF-α (infliximab, REMICADE^{®}), anti-integrin (namixilab) in the past 3 months.
* Subjects who are taking allopurinol, sulfasalazine, valerian, warfarin and corticosteroids, including budesonide and prednisone within 28 days prior to and throughout the study.
* Previous bowel resection, including prior colostomy, ileostomy or colectomy with ileorectal anastomosis.
* Symptomatic stenosis or ileal strictures; x-ray evidence of fibrosed bowel.
* Subjects with ulcerative colitis or short bowel syndrome.
* Subjects who present with, or with a history of, persistent intestinal obstruction, bowel perforation, uncontrolled GI bleed or abdominal abscess or infection, toxic megacolon.
* Subjects with fistulizing Crohn's disease or isolated small bowel Crohn's disease.
* Subjects with evidence of other serious infectious, autoimmune, hepatic, nephritic, or systemic disease or compromised organ function.
* Subjects with a history of GI tract malignancy or inflammatory bowel disease-associated malignant changes in the intestines.

### Primary Outcome Measures:

* Remission: defined as a CDAI of <150 (time frame: within 12 weeks).
* Response: defined as a fall in the CDAI by 100 points or more from baseline (time frame: within12 weeks).

### Secondary Outcome Measures:

* Reduction in Erythrocyte Sedimentation Rate ("ESR") levels Reduction in C-Reactive Protein ("CRP") levels,
   Increase in Inflammatory Bowel Disease Questionnaire ("IBDQ"), with score of ≥180 indicative of remission.

Each patient in the delayed release formulation group was given one daily oral dose of delayed release pharmaceutical composition containing 40 mg of 6-MP (Batch # 07C01RA, Expiry Date: 03/2010). Each patient in the reference group was given one daily oral doses of PURINETHOL^{®}, consisting of 3 x 50 mg 6MP tablets (total dose, 150 mg) (Lot #: A31737, expiry date: 10/2009). Due to the elevated LFT's shown, 2 of the 3 patients on the PURINETHOL had to reduce the dosage from 150 to 100 mg (2 x 50 mg tablets).

All patients were required to fill in a daily dosing diary. In addition, patients were required to fill in a daily CDAI questionnaire on each of the seven days before a scheduled clinic visit, every morning. Each patient served as his own control. The mean values (or changes in values) for all clinical and safety parameters for each visit were evaluated and comparison between baseline and last visit mean values were conducted.

CDAI scores were taken at every visit (t = 0, 2, 4, 6, 9, and 12 weeks), and IBDQ scores at baseline (t=0) and week 12. CDEIS scores were measured by colonoscopy at t = 0 and 12 weeks. Immunological testing, including IFN-γ ELISPOT of blood samples at baseline, weeks 9 and 12 and general inflammatory markers (CRP and ESR blood levels) at every visit (t = 0, 2, 4, 6, 9, and 12 weeks), were also conducted.

### Results:

Of the patients in the 6MP test group, five completed the study, while one patient in the Purinethol^{®} reference group completed the study. Study results presented below relate to these completed patients (i.e., PP, or per protocol population), the only patients for whom full data sets for comparison are available. Table 2 presents the CDAI scores for all PP patients, indicating the status of clinical response and/or remission after 12 weeks of treatment.

**Table 2. CDAI scores**

| Patient | Baseline | Week 2 | Week 4 | Week 6 | Week 9 | Week 12 |
|---|---|---|---|---|---|---|
| 1 | 220 | 150 (△ =70) | 73 (△ =147) | 151 (△ =69) | 48 (△ =172) | 42 (△ =178) |
| 4 | 339 | 260 (△ =79) | 325 (△ =14) | 346 (△ =-7) | 238 (△ =101) | 116 (△ =223) |
| 5 | 268 | 201 (△ =67) | 149 (△ =119) | 164 (△ =104) | 179 (△ =89) | 81 (△ =187) |
| 7 | 364 | 307 (△ =57) | 203 (△ =161) | 256 (△ =108) | 163 (△ =201) | 176 (△ =188) |
| 22 | 236 | 244 194 (△ =-8) | 193 (△ =43) | 194 (△ =42) | 181 (△ =55) | 160 (△ =76) |
| 010 (Purinethol^{®}) | 264 | 152 △ =132 | 102 △ =162 | 87 △ =177 | 102 △ =162 | 120 △ =144 |

| | | | | | | |
|---|---|---|---|---|---|---|
| △ : reduction from base line | | | | | | |

Table 2 above shows that the delayed release 6-MP test arm showed a gradual decrease in total CDAI score during the study. In the PP group, the average change from baseline was indicative of response (-169.0 ± 53.9). 80.0% of the delayed release 6-MP group achieved response, and 60.0% achieved remission, with one patient in remission as early as 2 weeks. The PURINETHOL^{®} reference arm also showed an overall decrease in CDAI (-144.0), with the one PP patient in the PURINETHOL^{®} reference arm achieving remission at 4 weeks.

Table 3 presents the CDEIS scores for all PP patients, indicating the status of mucosal healing after 12 weeks of treatment. The results below show that all patients who were administered the delayed release 6-MP pharmaceutical composition had lower total CDEIS scores after 12 weeks of treatment with once-daily delayed release 6-MP pharmaceutical composition. In the delayed release 6-MP test arm there was a decrease of about 50% while the single patient in the PURINETHOL^{®} reference arm showed no change in CDEIS score.

**Table 3. CDEIS scores**

| **Patient** | **Baseline** | **Week 12** | **Reduction** |
|---|---|---|---|
| 1 | 39 | 27 | 12 |
| 4 | 84.5 | 3 | 81.5 |
| 5 | 51 | 5 | 46 |
| 7 | 166 | 60 | 106 |
| 22 | 148 | 143 | 5 |
| 010 (Purinethol^{®}) | 50 | 50 | 0 |

The CDEIS improved for the delayed release 6-MP patients at levels of from 3% to 96% within 12 weeks. Mucosal healing was further corroborated by the colonoscopy report narratives, when comparing observations recorded at screening/baseline (pre-treatment) to the final colonoscopy observations recorded following 12 weeks of treatment. The CDEIS numeric scores above can be clearly correlated with the narrative findings described in Table 4 below:

**Table 4: Colonoscopy reports (baseline vs. 12 weeks)**

| | **Colonoscopy Report Findings** | |
|---|---|---|
| **Patient** | **Screening/Baseline (Pre-Treatment) Observations** | **Week 12 (Post-Treatment) Observations** |
| 01 | The colon was normal. The ileo-cecal valve was erythematous with superficial ulceration. 15 cm of terminal ileum were examined; deep serpiginous ulcerations in between normal mucosa were seen in the segment examined. | The colon is normal in appearance. Around the ileo-cecal valve, there were inflammatory changes (ulcerations/bleedings/edema). The terminal ileum was intubated up to 10 cm. The distal 2-3 cm were edematous and ulcerated. Proximally the appearance is normal. |
| 04 | Transverse colon: deep fissuring ulcerations, with cobblestone appearance, erythema and exudate. Ascending colon: similar in appearance to transverse colon, but milder degree of involvement and larger areas of normal mucosa interdispersed. Ileo-cecal valve: open with fish-mouth appearance, fibrotic and erythematous. Terminal ileum: A few apthous ulcerations in the distal 5 cm. | The macroscopic appearance of the colon is normal except for focal areas of mild erythema (no evidence of ulcerations). The ileo-cecal valve is open and has a fish-mouth appearance with some aphthous ulcerations surrounding it. The terminal ileum is normal in appearance. |
| 05 | In the proximal colon/cecum, active disease was noted with deep ulcerations, pseudopolyps and distortion of the architecture. In the rectum, several aphthas were noted. | Multiple pseudopolyps were seen, although at this time, there was no evidence for ulcerations or other inflammatory findings. The rest of the colon, including the rectum, were normal in appearance (no evidence for apthous ulcerations at this time in the rectum). |
| 07 | Terminal ileum looks normal. Ascending colon and cecum also normal From the anus till ascending colon, severe colitis, out of a short area of sigmoid (around 15 cm) that looks normal. Severe colitis with punch out ulcers and small, but mostly big ulcerations. | Terminal ileum, cecum and ascending colon appear normal. From the anus to the ascending colon there was moderate colitis (except for a short segment of the sigmoid which appears normal). 60% of the mucosa was mildly congested and erythematous with 30% of the area of the mucosa with pleomorphic ulcers and exudate. |
| | | |
| 022 | To the place of insertion the mucosa was congested. In addition, there were serpiginous ulcers. The mucosa has a cobble-stone appearance. Most of the colonic mucosa looks actively inflamed and with cobblestones and pseudopolyps; there were some sparing areas at the rectum, and ascending colon. | To the place of insertion the mucosa was inflammatory with mucopurulent exudate, and there were serpiginous ulcers. The mucosa has a cobble-stone appearance. Most of the colon was actively inflamed with pseudo-polyps, small islands of normal mucosa interspersed between deep ulcerations. The rectum and the distal transverse colon were relatively spared. |
| 010 (PURINETHOL^{®}) | Perianal disease is evident with multiple perianal fistuals drained with seton wires. In the distal rectum, a structuring was seen, with multiple aphthous ulcers and inflammatory changes (up to 3-4 cm from the anal verge). The rest of the colon was normal in appearance. The ileo-cecal valve was indurated, fibrous with a fishmouth appearance. Apthous ulceration were also seen in the distal 2 cm of the terminal ileum. Proximally the ileum is normal (examined up to 30 cm). | The endoscopic picture is identical to that reported in 6.02.07 (i.e., pre-treatment). Perianal disease was evident with multiple perianal fistulae drained with seton wires. In the distal rectum, a stricture is seen with inflammatory changes. The ileo-cecal valve was fixed-open. A few aptha were seen in the very distal terminal ileum and proximally to 3 cm, the TI appears normal. |

Thus, the results of the study show that the delayed release 6-MP pharmaceutical composition induced significant mucosal healing in the time frame measured (within 12 weeks), whereas conventional 6-MP therapy did not.

Table 5 below indicates that there was systemic immunological improvement, as evidenced by blood immunological markers, especially, levels of IFN-γ Elispot, measured at baseline (pre-treatment) and at post-treatment, weeks 9 and 12.

**Table 5. IFN-γ ELISPOT assay results**

| Patient | Baseline | Week 9 | Week 12 |
|---|---|---|---|
| 1 | 5.3 | 4 | 2.4 |
| 4 | 4.4 | 2.7 | 1 |
| 5 | 4 | 2.7 | 1.2 |
| 7 | 5 | 2 | 1.4 |
| 22 | 4 | 2.7 | 1.4 |
| 010 (Purinethol^{®}) | 5 | 1.7 | 1.3 |

Interferon activates other arms of the immune system such as macrophages and natural killer cells, and the decreased levels evident following treatment are indicative of decreased immune system reactivity. As noted in the table, immunological improvement was evident for all treatment groups. However, while it would be expected that reduction in systemic immunological activity would be seen following PURINETOL^{®} treatment, systemically administered, seeing such an effect following local delivery of test 6MP was an impressive finding.

Further evidence of the systemic effect on immunological and inflammatory markers, following local delivery of delayed release 6MP were the reductions in CRP and ESR levels measured at week 12 and compared to baseline levels, as depicted in Table 6 below:

**Table 6. CRP and ESR Levels**

| Parameter | Treatment | Baseline | Week 12 | △ : Reduction from base line |
|---|---|---|---|---|
| ESR | Delayed Release 6MP *N=5* | 34.6 ± 11.6 | 25.2±13.0 | -9.4±14.9 |
| | PURINETHOL^{®} *n=1* | 20 | 16 | -4.0 |
| CRP | Delayed Release 6MP *N=5* | 4.2± 2.5 | 2.4± 2.2 | -1.8 ± 2.8 |
| | PURINETHOL^{®} *n=1* | 1.4 | 0.8 | -0.6 |

The IBDQ questionnaire score, measuring improved quality-of-life, did not achieve remission (i.e., score greater than 180); nevertheless, improvement was seen in the greater increase in IBDQ score for the delayed release 6MP group (week 12 score of 164.8, an increase of 46.8 points relative to baseline), as compared to PURINETHOL^{®} (week 12 score of 141.0, an increase of 12 points relative to baseline).

As summarized in Table 7 below, data obtained for the delayed release 6-MP test group patients who completed the 12 week study demonstrated significant improvement in all three main efficacy parameters: local mucosal healing, clinical remission or response as measured by CDAI score, and systemic immunological status.

**Table 7: Delayed Release 6MP: Summary of Clinical and Immunological Improvement (% Change from Baseline)**

| Patient | Reduction in CDEIS within 12 weeks (%) | Reduction in CDAI within 12 weeks (%) | Reduction in IFN-γ ELISPOT within 12 weeks (%) |
|---|---|---|---|
| 1 | 30.76 | 49.09 | 54.71 |
| 4 | 96.44 | 65.78 | 77.27 |
| 5 | 90.19 | 69.77 | 70 |
| 7 | 63.85 | 51.64 | 72 |
| 22 | 3.37 | 32.2 | 65 |

| | | | |
|---|---|---|---|
| % was calculated compared to the baseline of each patient | | | |

Evidence of impressive local mucosal healing (CDEIS scores and colonoscopy report narratives), correlative with clinical response, and even remission, with CDAI scores below 150 as early as weeks 2 and 4, as well as underlying systemic immunological improvement (as reflected by IFN-γ ELISPOT levels) were clearly demonstrated for the delayed release 6-MP test group.

Side Effects: All three patients in the PURINETHOL^{®} group experienced adverse events associated with the drug. Two of them experienced clinically significant ALT (a liver enzyme) elevations attributed to PURINETHOL^{®}, of which one was able to complete the trial only after lowering the PURINETHOL^{®} dose and the other was forced to terminate the study. In contrast, only one patient in the delayed release group was forced to terminate the study due to global elevations in liver function tests.

Thus, evaluating the clinical, immunological and safety data from the study above, it appears that orally administered targeted ileal delayed release 6MP has benefit for Crohn's disease patients. Today, 6-MP is not considered part of the standard treatment to induce remission in Crohn's disease, mainly because of the long time it takes for it to show effect (3-4 months). However, the results of this study indicate that the present invention provides 6-MP formulations that have a role for remission induction in Crohn's disease patients, particularly those with mild to mild-moderate Crohn's disease.

### Example 2: Pharmacokinetic (PK) study of a delayed release composition according to the present invention

A PK study was conducted to determine the pharmacokinetic profiles (Cₘₐₓ, AUC and Tₘₐₓ), for the aforementioned clinical study.

Furthermore, immunology testing to measure the effect of the delayed release 6-MP pharmaceutical composition on immunological FACS analysis was performed on peripheral blood lymphocytes collected at 0 hour (pre-dosing), 12, and 24 hours following the administration of the delayed release 6-MP pharmaceutical composition, as compared to reference Purinethol^{®}. Lymphocytes were tested for surface marker expression including but not limited to: CD3+, CD4+, CD8+, NKT, LAP+, CD25+, FOXP3+, HLA DR, CD69, CD 127, and CD62.

A Phase I, pilot, randomized, single-dose, open label, 2-way, 2 period cross-over, comparative bioavailability study was conducted in 11 patients (males or non-pregnant females, 18-60 years of age at study entry) with Crohn's Disease (CDAI score <200 at entry), with or without adjunctive mesalamine treatment. Other drugs in the same class, which are normally given in much higher doses than mesalamine, such as sulfasalazine, were not allowed. The trial was conducted at the Hadassah Medical Center, Ein Kerem, Jerusalem, Israel.

Test drug: 1×40 mg delayed release 6-MP pharmaceutical composition (Batch number 07C01RAR).

Reference drug: 2×50 mg PURINETHOL^{®}*(Gates Pharmaceuticals,* Lot #: A31737).

The above doses were selected for the pharmacokinetic study since the 40 mg delayed release 6MP was previously shown in the clinical efficacy study above to be effective in inducing remission in patients with CD. The reference drug, 100 mg PURINETHOL ^{®} (administered as 2 x 50 mg PURINETHOL ^{®} tablets, since the 50 mg tablet is the only dosage form available), is a standard dose for off-label use of 6MP to treat CD, representative of and corresponding to roughly 2 mg/kg in patients, exactly mid-range of the 1.5-2.5 mg/kg recommendation for this drug.

Following a standardized meal, eligible subjects were told to fast until the next morning (at least 10 hours prior to dosing), and to continue to fast for another 4 hours following dosing. Water was allowed for up to 1 hour before and from 1 hour after dosing. Standardized meals/snacks were provided at 4, 8, 12, 14 and 24 hours post-dosing in each study period.

Both the test delayed release 6-MP pharmaceutical composition and the reference (PURINETHOL^{®}, 2 tablets together) were administered orally, once daily, and swallowed whole with 240 ml of water at room temperature. The subjects received their first treatment assignment at the first study period, and were crossed-over at the second study period to the alternative treatment arm.

For each of the study periods, 19 serial blood samples were collected per subject up to 24 hours post-dosing, for measurement of 6-MP levels. In addition, three additional blood samples were collected for immunology tests, 0 hour (pre-dosing) and 12 and 24 hours post dosing.

Analysis of the blood samples for 6-MP plasma levels was conducted via a validated LC/MS/MS method developed by Anapharm, Inc. in Canada, with an analytical range of 2-200 ng/ml. The pharmacokinetic analysis was performed using standard non-compartmental methods, with statistical analysis performed, using SAS® and 90% confidence interval and ratios for the relative mean In-transformed AUC₀₋ₜ, AUC_{0-inf}, and Cₘₐₓ of the test delayed release 6-MP and reference PURINETHOL^{®} formulations were calculated.

Immunological and statistical analyses of blood samples were performed at the laboratory of the Hadassah Hebrew University Medical Centers Liver Unit.

### Study Results - Pharmacokinetics:

### Reference drug (PURINETHOL^{®)}):

**Table 8. PK Data for PURINETHOL ^{®}**

| **Parameter** **(N=11)*** | **Mean** | **SD ±** |
|---|---|---|
| Cmax *(ng*/*l)* | 82.11 | 28.65 |
| Tmax *(h)* | 1.86 | 1.10 |
| AUC₀₋₂₄ *(ng-h*/*ml)* | 216.12 | 73.81 |
| AUC_{0-inf} *(ng-h*/*ml)* | 223.38 | 72.82 |

| | | |
|---|---|---|
| **Includes data from 11 patients* | | |

### Test drug (delayed release 6-MP):

**Table 9. PK Data for Delayed-Release 6MP**

| **Parameter** **(N=1)*** | **Value** | **SD ±** |
|---|---|---|
| Cmax *(ng*/*l)* | 6.14 | ------ |
| Tmax *(h)* | 9.00 | ------ |
| AUC₀₋₂₄ *(ng-h*/*ml)* | 10.24 | ------ |
| AUC_{0-inf} *(ng-h*/*ml)* | ----- | ------ |

| | | |
|---|---|---|
| **Includes data from 1 patient* | | |

At an analytical range for the 6-MP lower limit of quantitation (LLOQ) of 2.0 ng/ml, data could be presented for only 1 test subject, as noted above. However, if the analytical range is made more sensitive, with a lowered LLOQ (0.5 ng/ml), several other test patients would have also shown measurable, albeit negligible, 6-MP levels after 5-6 hours post dosing, with peak levels clustered about 8-9 hours, as tabulated below:

**Table 10. 6-MP levels measured, per patient, with LLOQ lowered to 0.5 ng/ml**

| **Time** **(h)** | **Patient** **#5** | **Patient** **#6** | **Patient** **#7** | **Patient** **#8** | **Patient** **#11** | **Patient** **#12** | **Patient** **#13** |
|---|---|---|---|---|---|---|---|
| 0 | -0.05 | 0.02 | -0.07 | 0.06 | 0.35 | 0.43 | 0.39 |
| 0.25 | -0.07 | 0.02 | -0.07 | 0.05 | 0.31 | 0.41 | 0.38 |
| 0.5 | -0.07 | 0.02 | -0.07 | 0.04 | 0.38 | 0.42 | 0.42 |
| 1 | -0.06 | 0.01 | -0.07 | 0.04 | 0.34 | 0.41 | 0.4 |
| 1.5 | -0.07 | 0.01 | -0.07 | 0.04 | 0.34 | 0.41 | 0.38 |
| 2 | -0.06 | 0.01 | -0.07 | 0.04 | 0.34 | 0.42 | 0.4 |
| 2.5 | -0.05 | 0.01 | -0.07 | 0.05 | 0.31 | 0.4 | 0.39 |
| 3 | -0.07 | 0.02 | -0.07 | 0.05 | 0.33 | 0.42 | 0.39 |
| 3.5 | -0.07 | 0.02 | -0.07 | 0.06 | 0.31 | 0.41 | 0.38 |
| 4 | -0.06 | 0.02 | -0.07 | 0.05 | 0.3 | 0.42 | 0.41 |
| 4.5 | -0.05 | 0.02 | -0.07 | 0.06 | 0.38 | 0.41 | 0.39 |
| 5 | -0.06 | 0.02 | -0.07 | 0.06 | 0.59 | 0.4 | 0.39 |
| 6 | -0.05 | 0.02 | -0.07 | 0.11 | 2.09 | 0.41 | 0.53 |
| 7 | 0.02 | 0.08 | -0.06 | 0.85 | 1.34 | 0.42 | 0.68 |
| 8 | 0.19 | 0.31 | -0.07 | 1.91 | 1.12 | 0.42 | 0.75 |
| 9 | 1.46 | 0.94 | -0.06 | 1.85 | 1.15 | 0.53 | 1.06 |
| 12 | 0.2 | 0.67 | 0.63 | 0.53 | 0.54 | 0.58 | 0.92 |
| 18 | -0.05 | 0.13 | 0.09 | 0.14 | 0.31 | 0.55 | 0.83 |
| 24 | -0.07 | 0.1 | -0.04 | 0.09 | 0.31 | 0.41 | 0.45 |

Data for the remaining 3 patients who received delayed release 6-MP and for whom blood samples were measured (patients #3, 4, and 10), did not show any measurable levels of 6-MP, even at the more sensitive LLOQ level at any time points measured.

### Study Results - Immunology:

After 12 hours, relative to 0 hour (pre-dose), a single dose of test drug increased the following T regulatory cells or T regulatory cell parameters on peripheral lymphocytes to a greater extent than PURINETHOL®:
- NKT cells (CD56)
- Activation markers (CD69, HLA DR)
- Memory cells (CD127)
- CD4/CD8 ratio
Increasing these T cell subsets, markers or receptors is indicative of improved immune response. The change in levels measured at 12 hours after a single dose of each treatment, relative to baseline, pre-treatment levels are tabulated below for various parameters for sample patients, with each patient acting as his own control.

**Table 11: Increases in NKT cells (CD56+): Sample Patient Data**

| Patient number | 6MP | | | PURINETHOL^{®} | | |
|---|---|---|---|---|---|---|
| | 0 hour | 12 hour | Delta (%) | 0 hour | 12 hour | Delta (%) |
| 03 | 5.94 | 6.1 | 6.82 | 9.94 | 6.3 | -36.62 |
| 05 | 2.38 | 3.58 | 50.42 | 13.04 | 6.19 | -52.53 |
| 07 | 3.7 | 3.79 | 2.43 | 13.53 | 6.03 | -55.43 |
| 12 | 8.71 | 13.82 | 58.67 | 10.39 | 11.09 | 6.74 |

**Table 12: Increases in CD127+: Sample Patient Data**

| Patient number | 6MP | | | PURINETHOL^{®} | | |
|---|---|---|---|---|---|---|
| | 0 hour | 12 hour | Delta (%) | 0 hour | 12 hour | Delta (%) |
| 04 | 15.99 | 21.71 | 35.77 | 22.36 | 15.25 | -31.80 |
| 06 | 9.89 | 17.91 | 81.09 | 2.27 | 1.08 | -52.42 |
| 08 | 12.04 | 14.5 | 20.43 | 1.11 | 1.27 | 14.41 |
| 09 | 3.19 | 3.58 | 12.23 | 12.5 | 7.45 | -40.40 |

2) After 12 hours, relative to 0 hour (pre-dose), a single dose of test drug decreased the following T regulatory cell parameter on peripheral lymphocytes to a greater extent than PURINETHOL^{®}:
• CD62L (adhesive proteins/selectins)
In this case, decreases in the cell adhesion protein, CD62L, is indicative of improved immune response. Immunohistochemistry studies of surgically resected specimens from patients with CD or ulcerative colitis have demonstrated that there is a statistically significant, nearly 4 fold increase in P-selectin immunoreactivity in the veins, venules and capillaries of highly inflamed gut compared to normal gut. This marked upregulation of P-selectin in inflamed tissue adversely affects normal lymphocyte localization and recirculation, interfering with normal immune response. The reduction in CD62L adhesion protein following a single dose of delayed release 6MP, as evidenced for the sampling of patients, as tabulated below, however, attests to the potential of the drug to decrease erratic leukocyte migration and restore immune homeostasis.

**Table 13: Decreases in levels of individual CD62L or CD62L complex.**

| Patient number | 6MP | | | PURINETHOL^{®} | | |
|---|---|---|---|---|---|---|
| | 0 hour | 12 hour | Delta (%) | 0 hour | 12 hour | Delta (%) |
| 07 | 0.22 | 0.05 | -77.27 | 0 | 0.13 | NA |
| 13 | 0.27 | 0 | -100.00 | 0 | 0 | NA |
| 04 | 41.77 | 41.09 | -1.63 | 48.92 | 61.72 | 26.17 |
| 05 | 0.06 | 0 | -100.0 | 0 | 0.04 | NA |
| 06 | 67.27 | 66.49 | -1.16 | 63.34 | 83.81 | 32.32 |
| 11 | 53.75 | 7.02 | -86.94 | 56.88 | 53.36 | -6.19 |

### Study Discussion and Conclusions:

The delayed release 6-MP pharmaceutical composition exhibits: (a) a much delayed Tₘₐₓ, with probable drug release in the distal small intestine and possibly beyond, and (b) negligible systemic drug levels, with a greatly reduced Cₘₐₓ and AUC observed. Since detectable levels could not be observed even at the much reduced LLOQ (theoretical 0.5 ng/ml) for all the subjects receiving the delayed release 6-MP pharmaceutical composition, it could be argued that the delayed release tablet formulation did not open, but passed through the body without release. However, the immunology data results observed in the study, as well as the clinical efficacy results observed in the previous study in which the exact same batch of delayed release 6-MP pharmaceutical composition was used, attest to the fact that the delayed release 6-MP pharmaceutical composition did, in fact, open and was released, following oral ingestion.

Furthermore, even after a single dose, the delayed release 6-MP pharmaceutical composition increased several T regulatory cells to a greater extent than PURINETHOL®. It decreased leukocyte migration and activating markers, and altered the CD4/CD8 lymphocyte ratio. The delayed release 6-MP pharmaceutical composition appeared to exert a profound systemic immunological effect.

## Claims

1. Use of 6-mercaptopurine for the manufacture of a medicament for treating mild to moderate inflammatory bowel disease, preferably Crohn's disease or ulcerative colitis, wherein the medicament is an oral delayed release pharmaceutical composition.

2. The use of claim 1, where the delayed release pharmaceutical composition releases 6-mercaptopurine after passage of the delayed release pharmaceutical composition through the stomach, where the delayed release pharmaceutical composition comprising 6-mercaptopurine comprises less than 50 mg of 6-mercaptopurine, is adapted to provide a plasma individual Cₘₐₓ of 6-mercaptopurine of less than 15 ng/ml and results in mucosal healing, clinical efficacy, improvement of immunological markers or a combination thereof.

3. The use of claim 1 or claim 2, where the delayed release pharmaceutical composition releases 6-mercaptopurine after passage of the delayed release pharmaceutical composition through the stomach, where the delayed release pharmaceutical composition comprising 6-mercaptopurine comprises less than 50 mg of 6-mercaptopurine, is adapted to provide an average plasma Cₘₐₓ of 6-mercaptopurine of less than 5% and results in mucosal healing, clinical efficacy, improvement of immunological markers or a combination thereof.

4. The use of claim 2, wherein the individual Cₘₐₓ of 6-mercaptopurine is of less than 10 ng/ml

5. The use of any one of claims 1-4, where the 6-mercaptopurine composition comprises about 40 mg of 6-mercaptopurine.

6. The use of any one of claims 1-5, where the delayed release pharmaceutical composition is administered once a day for at least 12 weeks.

7. The use of any one of claims 1-6, wherein mild to moderate inflammatory bowel disease is **characterized by** having a CDAI score about 220 or more, preferably 220 to about 400, before the treatment.

8. The use of any one of claims 1-7, where the delayed release pharmaceutical composition is administered to a patient who has not been treated with any steroid before the 6-mercaptopurine treatment.

9. The use of claim 8, where the delayed release pharmaceutical composition is administered to a patient who has not been treated with any steroid within 1 month before the 6-mercaptopurine treatment.

10. The use of any one of claims 8-9, where the delayed release pharmaceutical composition is administered to a patient who is not treated with any steroid during the 6-mercaptopurine treatment.

11. The use of any one of claims 1-10, where the delayed release pharmaceutical composition is administered to a patient who has not been treated with methotrexate, cyclosporine, anti TNF-α agent, or anti-integrin agent before the 6-mercaptopurine treatment, preferably within 3 months before the 6-mercaptopurine treatment.

12. The use of claim 11, where the delayed release pharmaceutical composition is administered to a patient who is not treated with methotrexate, cyclosporine, anti TNF-α agent, or anti-integrin agent during the 6-mercaptopurine treatment.

13. The use of any one of claims 1-12, where the delayed release pharmaceutical composition is administered to a patient at a dose of 140 mg/day or less, preferably less than 80 mg/day, most preferably less than 50 mg/day of 6-MP.

14. The use of any one of claims 1-13, wherein the dose of 6-mercaptopurine is about 1 to about 35 mg/day.

15. The use of any one of claims 1-14, where the dose of 6-mercaptopurine is administered one time per day.

16. The use of any one of claims 1-15, where the treatment results in a reduction of the CDEIS score, preferably the reduction is at least 10.

17. The use of any one of claims 1-16, where the treatment results in a reduction of the CDAI score from baseline, preferably the reduction is by 70 points or more from baseline within 12 weeks.

18. The use of any one of claims 1-17, where the reduction of the CDAI score is by 100 points or more from baseline within 12 weeks.

19. The use of any one of claims 1-18, where the treatment induces remission.

20. The use of claim 19, where the remission is induced within 12 weeks from the beginning of the treatment.

21. The use of any one of claims 1-20, where the treatment results in a significant improvement in the results of IFN-γ ELISPOT assay.

22. The use of any one of claims 1-21, where the treatment significantly reduces the size of intestinal/colonic ulcers and preferably also significantly reduces the number of ulcers in patients who are administered the delayed release pharmaceutical composition.

23. The use of any one of claims 1-22, wherein the treatment results in little or no liver function abnormalities in the patient.

24. The use of any one of claims 1-23, where the delayed release pharmaceutical composition comprising 6-mercaptopurine is in the form of a tablet, preferably in the form of enterically coated tablet.

25. The use of any one of claims 1-24, where the release of 6-mercaptopurine occurs after at least about 1 hour, preferably at least about 2, more preferably at least about 3 hours, after passage of the composition through the stomach.

26. The use of any one of claims 1-25, where the release of 6-mercaptopurine occurs after about 8 to about 9 hours after ingestion.

27. The use of any one of claims 1-26, wherein the treatment maintains remission.
